(19) Europäisches Patentamt
European Patent Office
Office européen des brevets

(11) Publication number: 0 266 849 B1

# (12) EUROPEAN PATENT SPECIFICATION

(45) Date of publication of patent specification: 13.03.91

(51) Int. Cl.5: C07B 45/00, C07C 327/26, C07D 211/58, C07D 207/22, C07D 279/16, C07D 209/88

(21) Application number: 87202150.6

(22) Date of filing: 05.11.87

## (54) Process for introducing a thiocarboxylic ester group at the ortho-position of phenols or phenylamines.

(30) Priority: 06.11.86 JP 264658/86

(43) Date of publication of application:
11.05.88 Bulletin 88/19

(45) Publication of the grant of the patent:
13.03.91 Bulletin 91/11

(84) Designated Contracting States:
AT BE CH DE ES FR GB GR IT LI LU NL SE

(56) References cited:

W. THEILHEIMER: "Synthetic Methods of Organic Chemistry", vol. 27, 1973, page 273, S. KARGER, Basel, CH;

JOURNAL OF THE AMERICAN CHEMICAL SOCIETY, vol. 45, July 1923, pages 1744-1752, R.J. KAUFMANN et al.: "Production of imido thiol esters by the condensation of thiocyanates with resorcinol or phloroglucinol"

(73) Proprietor: SHIONOGI SEIYAKU KABUSHIKI KAISHA trading under the name of SHIONOGI & CO. LTD.
1-8, Doshomachi 3-chome Chuo-ku
Osaka 541(JP)

(72) Inventor: Adachi, Makoto
2285-6 Ichihara Heguri-cho
Ikoma-gun Nara(JP)
Inventor: Matsumura, Hiromu
15-10-839, Asahigaoka-cho
Ashiya-shi Hyogo(JP)
Inventor: Sugasawa, Tsutomu
5-21-19, Mikageyamate Higashinada-ku
Kobe-shi Hyogo(JP)

(74) Representative: Bruin, Cornelis Willem et al
OCTROOIBUREAU ARNOLD & SIEDSMA
Sweelinckplein 1
NL-2517 GK The Hague(NL)

EP 0 266 849 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a process for introducing a thiocarboxylic ester group at the ortho-position of phenols or phenylamines. More particularly, this invention is directed to a process for introducing a thiocarboxylic ester group at the ortho-position of phenols or phenylamines which have been found to be useful in the synthesis of medicinals, pesticides and dyes.

The prior art comprised already the following reactions:

a) A reaction described by:

R. J. Kaufmann et al.in J. Am. Chem. Soc. 45 1744 (1923)

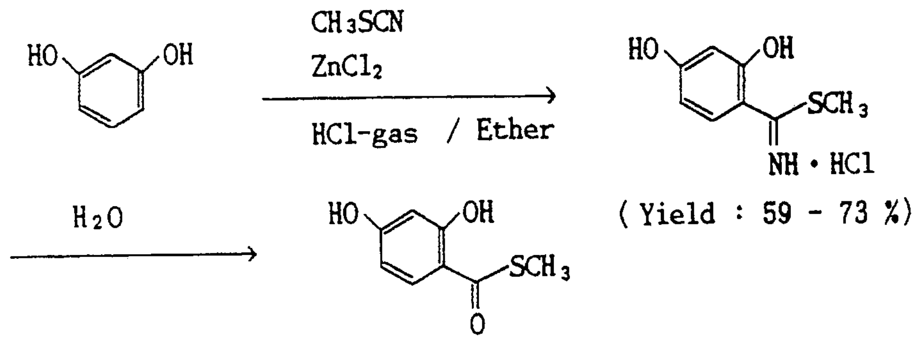


This reaction gives an objective compound only by using polyhydric phenols. When simple phenols are subjected to this reaction, however, no ortho- is substituted product is obtained.

b) A reaction described by:

Dow Chemical in US-A-3338947 and US-A-3377372:

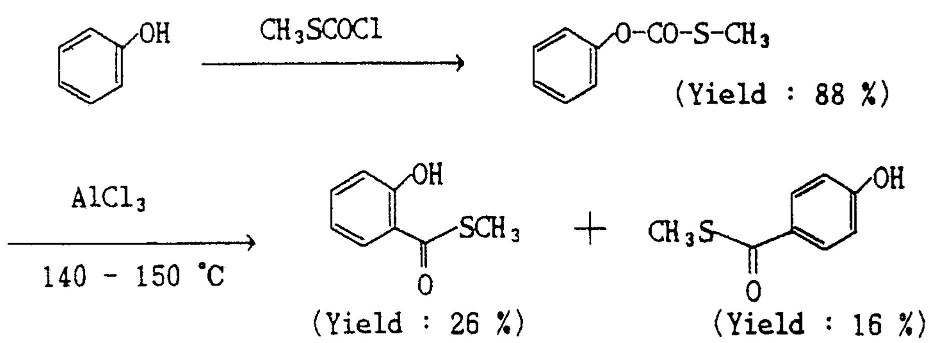


c) A reaction described by:

S. Ohta et al. in Tetrahedron Lett., 22, 3245 (1981):

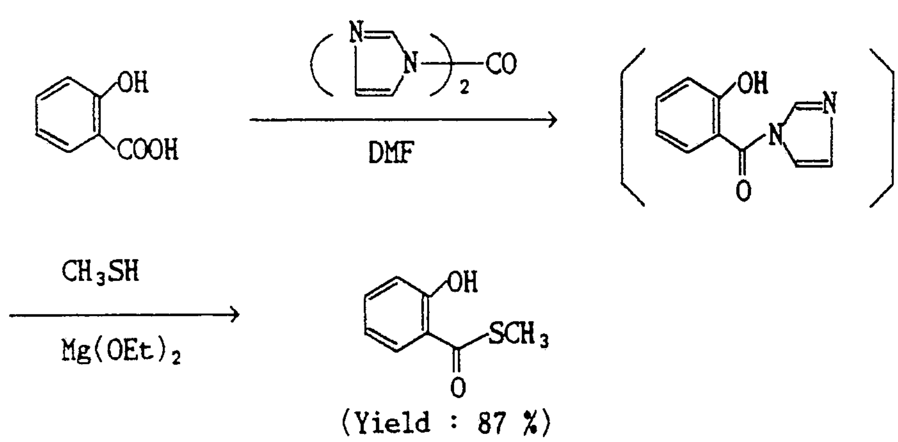

These conventional reactions do not always give satisfactory yields, and ortho-substituted thiobenzoic acid ester derivatives having another desirable substituent at any desired position cannot be obtained.

According to the present invention, there is provided a process for introducing a thiocarboxylic ester group at the ortho-position of phenols or phenylamines which process is characterized by reacting a phenyl compound having a hydroxy group or an optionally substituted amino or cyclic amino group, of which at least one ortho-position is vacant, with a $C_1$- $C_5$ alkyl thiocyanate, $C_7$-$C_{13}$ aralkyl thiocyanate or $C_6$-$C_{12}$ aryl thiocyanate in the presence of boron trichloride or tribromide and hydrolyzing the resulting boron-containing intermediate product under acidic conditions to give a desired end product.

Said process is illustrated by the undermentioned overall reaction scheme:

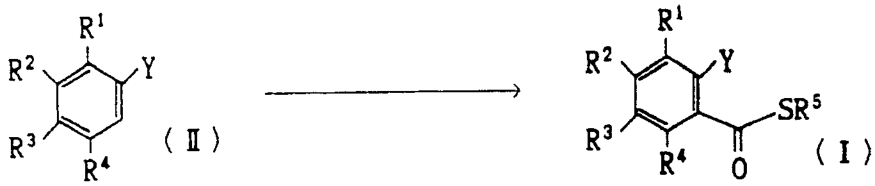

wherein $R^1$, $R^2$, $R^3$ and $R^4$ each are hydrogen, halogen, $C_1$ - $C_5$ alkyl, $C_1$ - $C_5$ alkoxy, $C_1$ - $C_5$ alkylthio,$C_6$-$C_{12}$ aryloxy, $C_7$ - $C_{15}$ aralkyl, $C_7$ - $C_{15}$ aralkoxy or $C_1$ - $C_{10}$ acylamino, or ($R^1$ and $R^2$) or ($R^2$ and $R^3$) taken together form a condensed benzene ring optionally substituted by halogen or $C_1$ - $C_5$ alkyl or $C_1$ - $C_5$ alkoxy, Y is hydroxy, amino or -NHR, and R is $C_1$ -$C_5$ alkyl $C_7$ - $C_{13}$ aralkyl, $C_6$ - $C_{12}$ aryl or N-(methyl- or benzyl-substituted)-aza($C_3$ - $C_7$ cycloalkyl), and $R^5$ is $C_1$ - $C_5$ alkyl or $C_7$ - $C_{13}$ aralkyl or $C_6$ - $C_{12}$ aryl.) . In some cases, NHR and $R^1$ taken together will form a 5- or 6-membered heterocyclic ring B condensed with the initial benzene ring A and represented by the formula:

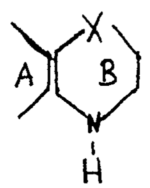

(IV)

in which X is a single bond or $CH_2$, 0, S or N(methyl)-and the B ring may optionally be condensed with a benzene, pyridine or cyclohexane ring which may optionally be substituted by halogen or $C_1$-$C_5$ alkyl or $C_1$-$C_5$ alkoxy. Further, the aforesaid alkyl, alkoxy, aryl, aryloxy, aralkyl and aralkoxy radicals may optionally be substituted by $C_1$-$C_5$-alkyl, $C_1$-$C_5$-alkoxy or halogen.

Among the starting phenyl compounds (II) two types A and B (phenylamines of formula IIa and phenols of formula IIb) may be distinguished. The reactions with these starting compounds will proceed in accordance with the following reaction schemes:

A type

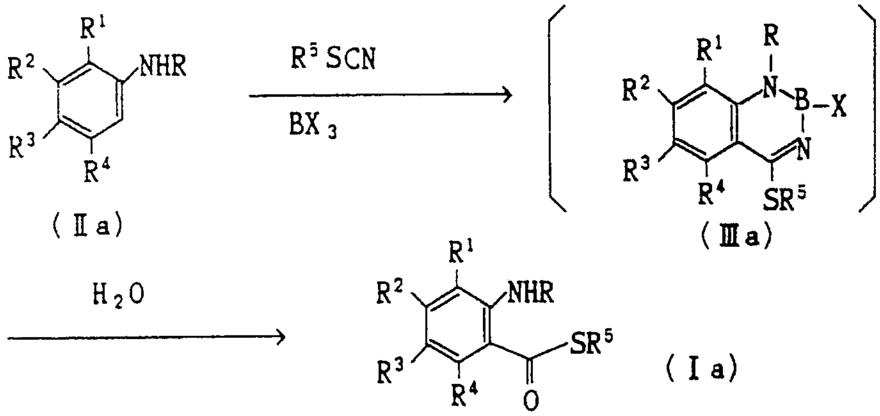


( IIa ) ( IIIa ) ( I a)

B type

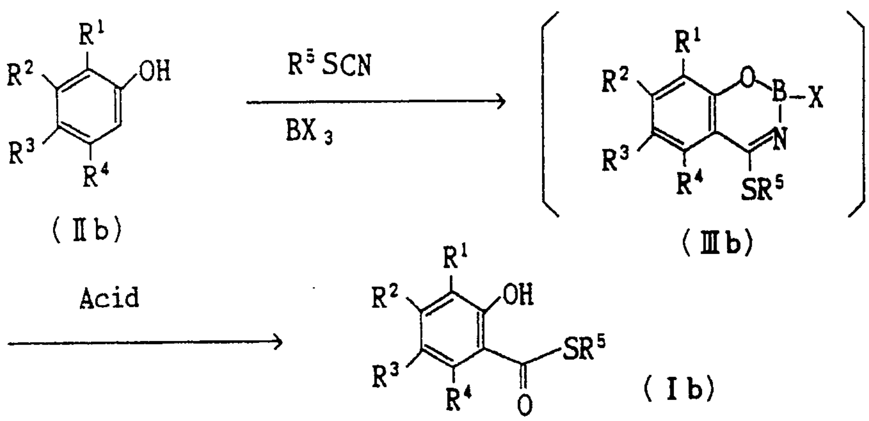


( IIb ) ( IIIb ) ( I b)

wherein $R^1$, $R^2$, $R^3$, $R^4$ and R have the same meanings as defined above, and X is Cl or Br , $R^5$ is $C_1$ - $C_5$ alkyl, $C_7$ - $C_{13}$ aralkyl or $C_6$ -$C_{12}$ aryl.

Further two types C and D of starting compounds may be distinguished as special subtypes of type A. These starting compounds wherein NHR and $R^1$ of formula II have formed together a 5- or 6-membered heterocyclic ring, can be represented by formulae IIc and IId. The reactions with them will proceed in accordance with the following reaction schemes:

C type

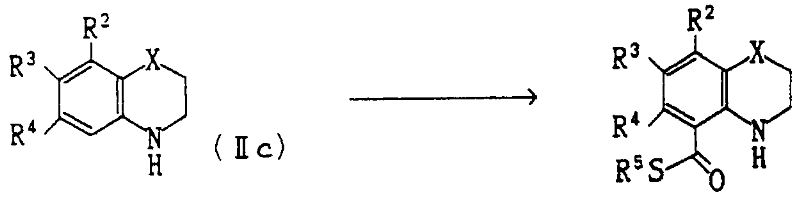

( II c) ( I c)

D type

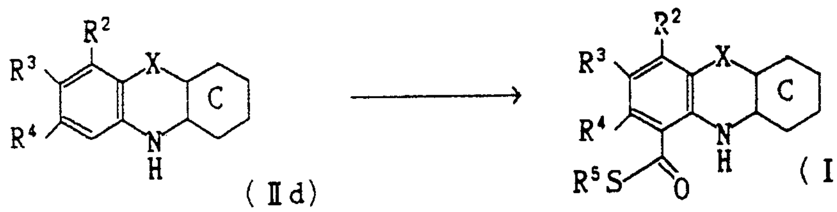

( II d) ( I d)

wherein the ring C is a benzene, pyridine or cyclohexane ring, each of which is optionally substituted by halogen, $C_1$ - $C_5$ alkyl or $C_1$ - $C_5$ alkoxy, X is a single bond, or $CH_2$ O, S or N(methyl)-, and $R^2$, $R^3$, $R^4$ and $R^5$ have the same meanings as defined above

The terms used in the above definitions are illustratively explained below.

The term "$C_1$ - $C_5$ alkyl" herein employed refers to a straight or branched saturated aliphatic hydrocarbon radical such as methyl, ethyl, n-propyl, n-butyl, isobutyl, sec-butyl, tert-butyl, n-pentyl, isopentyl, sec-pentyl, tert-pentyl and neo-pentyl.

The term "$C_1$ - $C_5$ alkoxy" represents methoxy, ethoxy, n-propoxy, isopropoxy, n-butoxy, isobutoxy, sec-butoxy, tert-butoxy, neo-pentyloxy and sec-pentyloxy.

The term "$C_1$ - $C_5$ alkylthio" represents methylthio, ethylthio, propylthio, isopropylthio, butylthio, pentylthio and isopentylthio.

The term "$C_6$ - $C_{12}$ aryl" means phenyl, tolyl, xylyl, naphthyl, pyridyl, thienyl and furyl.

The term "$C_6$ - $C_{12}$ aryloxy" means phenoxy, naphthoxy, pyridyloxy and tolyloxy.

The term "$C_7$ - $C_{15}$ aralkyl" includes benzyl, phenethyl, phenylpropyl, phenylbutyl, naphthylmethyl and naphthylpropyl.

The term "$C_7$ - $C_{15}$ aralkoxy" includes benzyloxy, phenethyloxy, phenylpropyloxy, phenylbutyloxy and naphthylmethoxy.

The term "$C_1$ - $C_{10}$ acylamino" includes formylamino, acetylamino, propionylamino, butyrylamino, benzoylamino, phenylacetylamino and phenylbutyrylamino.

Moreover,the above mentioned alkyl, alkoxy, aryl, aryloxy, aralkyl and aralkoxy radicals may be optionally substituted by $C_1$ - $C_5$ alkyl,$C_1$ - $C_5$ alkoxy or halogen.

The thiocyanate usable as one of the other starting materials includes alkyl thiocyanates, aralkyl thiocyanates and aryl thiocyanates, and the hydrocarbon groups thereof may have one or more substituents selected from alkyl, alkoxy and halogen. Examples of the thiocyanates used herein, are alkyl thiocyanates such as methyl thiocyanate, ethyl thiocyanate, propyl thiocyanate or butyl thiocyanate, aralkyl thiocyanates such as benzyl thiocyanate or phenethyl thiocyanate and aryl thiocyanates such as phenyl thiocyanate or naphthyl thiocyanate.

The process of the present invention as applied to starting materials of the A-type, B-type, C-type or D-type is explained below in more detail.

A type

The phenylamines (II a) are allowed to react with thiocyanates in the presence of boron trichloride or boron tribromide to give boron -containing intermediate products (IIIa) and such intermediate

products (IIIa) are then hydrolyzed with water to give the objective compounds (Ia).

The reaction of phenylamine (IIa) with thiocyanate is performed in the presence of boron trichloride or tribromide in an appropriate inert solvent (e.g. methylene chloride, 1,2-dichloroethane, benzene, toluene, xylene) at room temperature (1-30°C) or at a temperature from room temperature to about the boiling point of the solvent used. The hydrolysis can be performed in an aqueous solution at ordinary temperature (0 -30° C) or under heating ( 30 - 100 °C).

B type

The phenols (II b) are allowed to react with thiocyanates in the presence of boron trichloride or boron tribromide to give boron-containing intermediate products (IIIb) and such intermediate products (IIIb) are hydrolyzed thereupon with water to give the objective compounds (Ib). The reaction of phenols (II b) with thiocyanates can be performed as described for the A type of starting materials. The reaction proceeds smoothly, but the yield can be increased by adding still another Lewis acid (e.g. aluminium chloride, tin tetrachloride, titanium tetrachloride) to the reaction mix-ture. Then the resulting intermediate product is hydrolyzed with dilute acid (e.g. hydrochloric acid, sulfuric acid, etc.) to give the objective compound (I b). The hydrolysis proceeds in a conventional manner. The hydroxy group of the thiocarboxylic esters (I b) obtained in this reaction can be easily subjected to alkylation with alkyl halides, aryl halides, aralkyl halides, dialkyl sulfates or epihalohydrins in a conventional manner.

C type and D type

The cyclic amines (II c) and (II d) can be allowed to react with thiocyanates in the same way as in the case of phenylamines (II a).

Presently preferred and practical embodiments of the process of the present invention are illustratively shown in the following working examples, which do not limit the technical scope of the invention.

The abbreviations used in this specification have the following meanings.

| | | |
|---|---|---|
| Me : methyl | n-Pr : n-propyl | n-Bu : n-butyl |
| Et : ethyl | i-Pr : isopropyl | Ph : phenyl |
| OMe : methoxy | SMe : methylthio | $CH_2Ph$ : benzyl |
| BN : benzene | DM : methylene chloride | |
| TL : toluene | DE : 1,2-dichloroethane | |
| DMF : dimethylformamide | | |

Example 1

To a solution of 1.07 g of N-methylaniline in 10 ml of toluene was added 5.5 ml of a solution of 2.02 M boron trichloride in toluene under ice-cooling, and the mixture was refluxed with heating on an oil bath for 1 hr. and concentrated under atmospheric pressure. The residue was cooled with ice-water, mixed with 1 ml of methyl thiocyanate and stirred for 30 min. under ice-cooling and 2.5 hr. at room temperature. The reaction mixture was mixed with 20 ml of water and heated under reflux for 30 min. The product was extracted with toluene, dried over anhydrous magnesium sulfate, concentrated and subjected to chromatography on silica gel. The eluate with toluene was concentrated to give 1.62 g of N-methylthioanthranilic acid S-methyl ester as a pale yellow oil.

Yield : 90 %

IR : 3360, 1626 $cm^{-1}$

Example 2 - 15

The process of Example 1 was repeated with other starting materials of formula IIa in order to obtain the corresponding objective compounds of formula Ia, according to the following reaction scheme:

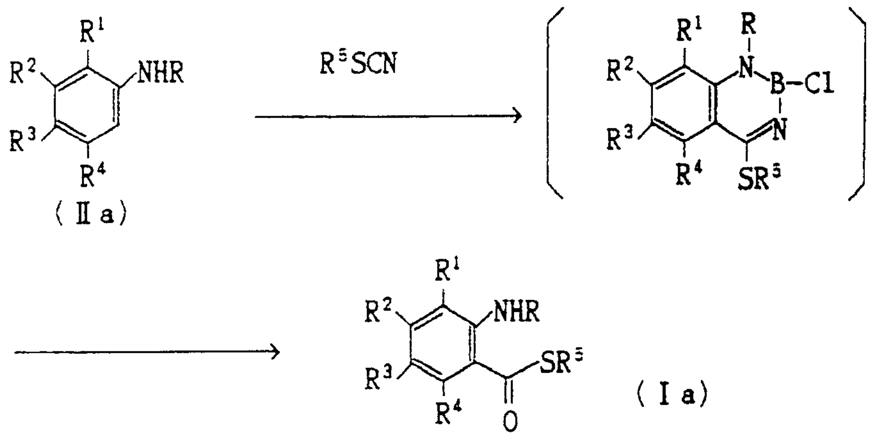


(IIa)

(Ia)

wherein $R^1$, $R^2$, $R^3$, $R^4$, $R^5$ and R have the same meanings as defined above.

The reaction was performed at room temperature. Reaction conditions and results as well as the nature of starting materials and end products are shown in Table 1.

Table 1

| Ex No | $R^1$ | $R^2$ | $R^3$ | $R^4$ | $R^5$ | R | Time (hr) | m.p. (°C) or IR ($cm^{-1}$) | Yield (%) |
|---|---|---|---|---|---|---|---|---|---|
| 2 | H | H | H | H | Et | Me | 3 | 1622 | 73 |
| 3 | H | H | H | H | n-Bu | Me | 3 | 1625 | 87 |
| 4 | H | H | H | H | $CH_2Ph$ | Me | 3 | 1623 | 87 |
| 5 | H | H | H | H | Me | Et | 15 | 1620 | 79 |
| 6 | H | H | H | H | Me | n-Pr | 15 | 1626 | 76 |
| 7 | H | H | H | H | Me | n-Bu | 15 | 28 - 29 | 76 |
| 8 | H | H | H | H | Me | $CH_2Ph$ | 15 | 47 - 48 | 91 |
| 9 | H | Cl | H | H | Me | Et | 24 | 83 - 84 | 45 |
| 10 | H | Cl | H | H | i-Pr | Et | 24 | 1620 | 46 |
| 11 | H | H | Cl | H | Me | Et | 65 | 60 - 61 | 68 |
| 12 | H | F | H | H | Me | Et | 24 | 51 - 52 | 54 |
| 13 | H | H | F | H | Me | Et | 65 | 50 - 51 | 64 |
| 14 | H | Cl | Cl | H | Me | Et | 65 | 84 - 85 | 21 |
| 15 | H | OMe | H | H | Me | Et | 3 | 55 - 56 (4-OMe Compd.)<br>1608 (6-OMe Compd.) | 49<br>23 |

Example 16

To a solution of 1.14 g of N-(1-methyl-4-piperidinyl)-aniline in 20 ml of toluene was added 3.6 ml of a solution of 2.04 M boron trichloride in toluene and the mixture was refluxed with heating on an oil bath for 2 hr. After cooling, the reaction mixture was mixed with 0.49 ml of methyl thiocyanate and refluxed with heating on an oil bath for 2 hr. After cooling, 20 ml of water was added and the mixture was stirred at 110 °C on an oil bath for 1 hr. The reaction mixture was turned to basic conditions with aqueous 2 N sodium carbonate and extracted with toluene. The toluene layer was dried over anhydrous magnesium sulfate, chromatographed on a column of 20 g of aluminium oxide for purification, eluted with methylene chloride and concentrated. The crystalline residue (1.35 g) was recrystallized from ether - petroleum ether to give 1.21 g of N-(1-methyl-4-piperidinyl)thioanthranilic acid S-methyl ester melting at 64 - 65 °C as yellow crystals.

Yield : 76 %

Anal Calcd. for $C_{14}H_{20}ON_2S$

: C, 63.60; H, 7.60; N, 10.60; S, 12.13

Found (%) : C, 63.56; H, 7.69; N, 10.57; S, 11.92

Example 17 - 23

The process of Example 16 was repeated with other starting materials of formula IIa in order to obtain the corresponding objective compounds of formula $Ia^1$ in accordance with the following reaction scheme:

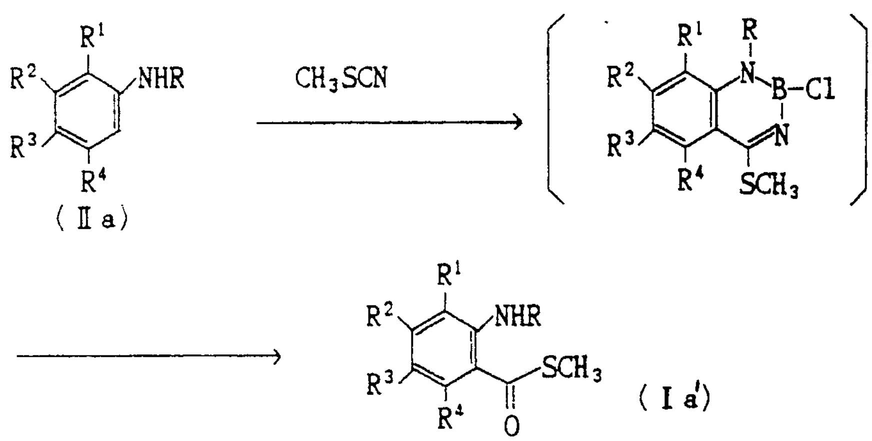


( II a)

( I a')

wherein $R^1$, $R^2$, $R^3$, $R^4$ and R have the same meanings as defined above.

The reaction was performed under refluxing conditions. Further conditions and results as well as the nature of starting materials and end products are shown in Table 2.

Table 2

| Ex. No. | R¹ | R² | R³ | R⁴ | R | Solvent | Time (hr) | m.p. (°C) | Yield (%) |
|---|---|---|---|---|---|---|---|---|---|
| 17 | H | H | Cl | H | 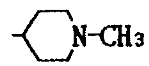 | TL | 2 | 81 - 82 | 80 |
| 18 | H | H | F | H | 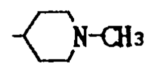 | TL | 2 | 68 - 69 | 66 |
| 19 | H | H | OMe | H | 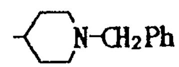 | DE | 3 | 111-113 | 76 |
| 20 | H | H | H | H | 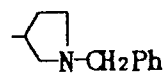 | TL | 2 | 187-188* | 81 |
| 21 | H | H | Cl | H | 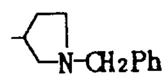 | TL | 2 | 157-158* | 61 |
| 22 | H | F | H | H | 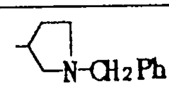 | TL | 2 | 179-181* | 77 |
| 23 | H | H | OMe | H | 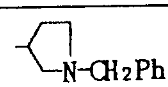 | DE | 3 | 125-127* | 80 |

※ : HBr salt

Example 24

To a solution of 1.69 g of diphenylamine in 10 ml of toluene was added 5.5 ml of a solution of 2.02 M boron trichloride in toluene under ice-cooling, and the mixture was refluxed with heating on an oil bath for 1 hr. After ice-cooling, the reaction mixture was mixed with 1 ml of methyl thiocyanate and stirred at room temperature for 16 hr. The reaction mixture was mixed with 20 ml of water and refluxed with heating on an oil bath for 30 min. The insoluble materials were filtered off and the toluene layer was dried over anhydrous magnesium sulfate, chromatographed on a column of silica gel and eluted with toluene. The solvent was concentrated to give 1.03 g of N-phenylthioanthranilic acid S-methyl ester as a yellow oil.

Yield : 42 %

IR (film) : 3300, 1628 $cm^{-1}$

Example 25 - 26

The process of Example 24 was repeated with other starting materials of formula $IIc^1$ in order to obtain the corresponding objective compounds of formula $Ic^1$ according to the following reaction scheme:

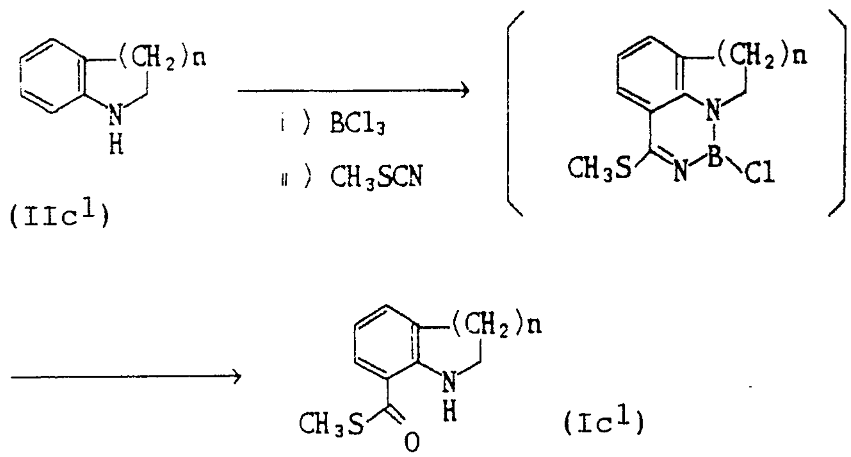


($IIc^1$)

($Ic^1$)

wherein n is a integer of 1 or 2 . Reation conditions and results are shown in Table 3.

Table 3

| Ex. No. | n | Temperature (°C) | Time (hr) | m.p. (°C) or IR (film) | Yield (%) |
|---|---|---|---|---|---|
| 25 | 1 | room temperature | 3 | 85 - 86 | 82 |
| 26 | 2 | room temperature | 3 | 1620 $cm^{-1}$ | 85 |

Example 27

To a solution of 1.35 g of 3,4-dihydro-2H-1,4-benzoxazine in 10 ml of 1,2-dichloroethane was added 5 ml of a solution of 2.02 M boron trichloride in 1,2-dichloroethane and a mixture solution of 0.82 ml of methyl thiocyanate, 2.9 ml of tri-n-butylamine and 10 ml of 1,2-dichloroethane under ice-cooling. The resulting mixture was stirred at room temperature for 4 hr. The reaction mixture was mixed with 5 ml of 2 N HCl and 40 ml of water, and heated at 110 °C on an oil bath to evaporate 1,2-dichloroethane and further stirred for 1 hr. The product was extracted with toluene, dried over anhydrous magnesium sulfate, chromatographed on a column of 10 g of silica gel and eluted with methylene chloride. The solvent was concentrated to give 1.60 g of 3,4-dihydro-2H-1,4-benzoxazin-5-thiocarboxylic acid S-methyl ester as a yellow oil.

Yield : 76 %

IR (film) : 3350, 1622 $cm^{-1}$

Example 28 - 29

The process of Example 27 was repeated with other starting materials of formula $IIc^2$ in order to obtain the corresponding objective compounds of formula $Ic^2$ in accordance with the following reaction scheme:

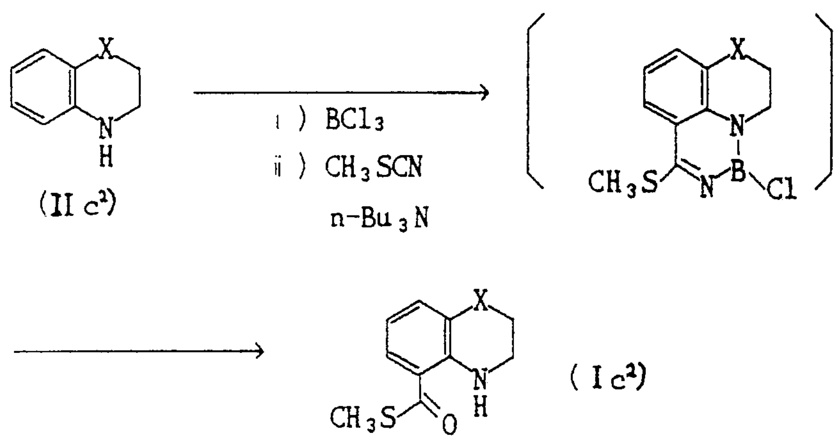


(II c²)

(I c²)

wherein X has the same meaning as defined above. Reaction conditions and results are shown in Table 4.

Table 4

| Ex. No. | X | Temperature (°C) | Time (hr) | IR (film) $cm^{-1}$ | Yield (%) |
|---|---|---|---|---|---|
| 28 | S | room temperature | 16 | 3325, 1625 | 58 |
| 29 | N-Me | room temperature | 17 | 3340, 1619 | 49 |

Example 30

To a solution of 1.67 g of carbazole in 10 ml of toluene was added 5.4 ml of a solution of 2.04 M boron trichloride -toluene under ice-cooling, and the mixture was refluxed with heating on an oil bath for 1 hr. After ice-cooling, the mixture was mixed with 1 ml of methyl thiocyanate, stirred at room temperature for 3 hr., mixed with 30 ml of water and refluxed with heating on an oil bath for 1 hr. The product was extracted with toluene, dried over anhydrous magnesium sulfate, chromatographed on a column of 10 g of silica gel and eluted with toluene. The eluate was concentrated to give 2.16 g of the crude crystals. Recrystallization from methylene chloride - ether gave 2.08 g of carbazol-1-thiocarboxylic acid S-methyl ester melting at 122 - 123 °C as crystals.

Yield : 86 %

Anal Calcd. (%) for $C_{14}H_{11}ONS$

: C, 69.68; H, 4.60; N, 5.81; S, 13.29

Found (%) : C, 69.77; H, 4.59; N, 5.79; S, 13.21

Example 31

Using 1.99 g of phenothiazine as a starting material, the process of Example 30 was repeated. As a result,

0.798 g of phenothiazin-1-thiocarboxylic acid S-methyl ester was obtained as crystals melting at 92 - 93 °C.

Yield : 29 %

Anal Calcd. (%) for $C_{14}H_{11}ONS$

: C, 61.51; H, 4.06; N, 5.12; S, 23.46

Found (%) : C, 61.73; H, 3.92; N, 5.12; S, 23.28

Example 32

To 5.5 ml of a solution of 2.2 M boron trichloride in 1,2-dichloroethane were added a solution of 941 mg of phenol in 10 ml of 1,2-dichloroethane and 0.82 ml of methyl thiocyanate and 1.33 g of aluminium chloride under ice-cooling. After dissolving the aluminium chloride with stirring at room temperature, the mixture was heated at 80 °C on an oil bath for 3 hr. After ice-cooling, the reaction solution was mixed with 6 g of ice and 15 ml of 2 N HCl and heated at 110 °C on an oil bath under stirring to evaporate 1,2-dichloroethane and further stirred for 1 hr. After cooling, the mixture was extracted with toluene. The toluene layer was dried over anhydrous magnesium sulfate and chromatographed on a column of 20 g of silica gel for purification. The eluate with toluene was concentrated to give 1.47 g of 2-hydroxy-thiobenzoic acid S-methyl ester as a colorless oil.

Yield : 88 %

IR (film) : 1628 $cm^{-1}$

Example 33 - 45

The process of Example 32 was repeated with other starting materials of formula IIb in order to obtain the corresponding compounds of formula Ib according to the following reaction scheme:

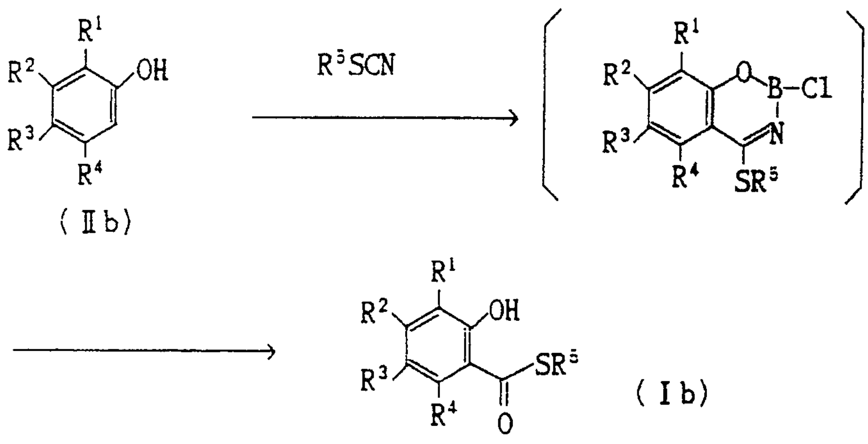


(IIb)

(Ib)

wherein $R^1$, $R^2$, $R^3$, $R^4$ and $R^5$ have the same meanings as defined above.

The reaction was performed with boron trichloride and aluminium chloride in l,2-dichloroethane at 80° C. Other reaction conditions and results as well as the nature of starting materials and end products are shown in Table 5.

Table 5

| Ex. No. | $R^1$ | $R^2$ | $R^3$ | $R^4$ | R | Time (hr) | m.p. (°C) or IR (film) | Yield (%) |
|---|---|---|---|---|---|---|---|---|
| 33 | H | H | H | H | Et | 3 | 1630 $cm^{-1}$ | 75 |
| 34 | H | H | H | H | n-Bu | 3 | 1621 $cm^{-1}$ | 62 |
| 35 | Me | H | H | H | Me | 3 | 1622 $cm^{-1}$ | 79 |
| 36 | H | Me | H | H | Me | 3 | 72 - 73 | 68 |
| 37 | H | H | Me | H | Me | 3 | 1637 $cm^{-1}$ | 96 |
| 38 | H | Me | Me | H | Me | 3 | 79 - 80 | 85 |
| 39 | Cl | H | H | H | Me | 64 | 89 - 90 | 25 |
| 40 | H | Cl | H | H | Me | 16 | 70 - 71 | 79 |
| 41 | H | H | Cl | H | Me | 64 | 90 | 40 |
| 42 | Cl | Cl | H | H | Me | 64 | 93 - 94 | 29 |
| 43 | Cl | H | H | OMe | Me | 1 | 98 - 99 | 41 |
| 44 | H | H | SMe | H | Me | 1 | 45 - 46 | 13 |
| 45 | Me | H | Cl | OMe | Me | 2 | 100 - 101 | 21 |

Example 46

To a solution of 1.13 ml of boron tribromide in 6 ml of 1,2-dichloroethane were added a solution of 941 mg of phenol in 10 ml of 1,2-dichloroethane, as well as 0.82 ml of methyl thiocyanate and 1.33 g of aluminium chloride under ice-cooling. After dissolving aluminium chloride with stirring at room temperature, the mixture was heated at 80 °C on an oil bath for 3 hr. After ice-cooling, the reaction solution was mixed with 6 g of ice and 15 ml of 2 N HCl and heated at 110 °C on an oil bath under stirring to evaporate 1,2-dichloroethane and further stirred for 1 hr. After cooling, the mixture was extracted with toluene. The toluene layer was dried over anhydrous magnesium sulfate and chromatographed on a column of 20 g of silica gel for purification. The eluate with toluene was concentrated to give 1.41 g of 2-hydroxythiobenzoic acid S-methyl ester as a colorless oil.

Yield : 84 %

Example 47

To 5 ml of a solution of 2.02 M boron trichloride - benzene were added a solution of 1.24 g of 3-methoxyphenol in 12 ml of benzene, as well as 0.82 ml of methyl thiocyanate and 1.33 g of aluminium chloride under ice-cooling. After stirring at room temperature for 16 hr., the reaction mixture was cooled with ice water, mixed with 5 g of ice and 15 ml of 2 N HCl and stirred with heating at 110 °C on an oil bath to evaporate 1,2-dichloroethane and further stirred for 2 hr. After cooling, the mixture was extracted with toluene. The toluene layer was dried over anhydrous magnesium sulfate and chromatographed on a column of 20 g of silica gel for purification. The product (1.48 g) eluted was recrystallized from ether - petroleum ether to give 1.33 g of 2-hydroxy-4-methoxythiobenzoic acid S-methyl ester melting at 56 - 57 °C as white needles.

Yield : 67 %

Anal Calcd. (%) for $C_9H_{10}O_3S$ :

C, 54.52, H, 5.08, S, 16.14

Found (%) : C, 54.34, H, 5.15, S, 16.24

IR ($CHCl_3$) : 3220, 1631 $cm^{-1}$

Example 48 - 51

The process of Example 47 was repeated with other starting materials of formula IIb in order to obtain the corresponding objective compounds ($Ib^1$) according to the following reaction scheme:

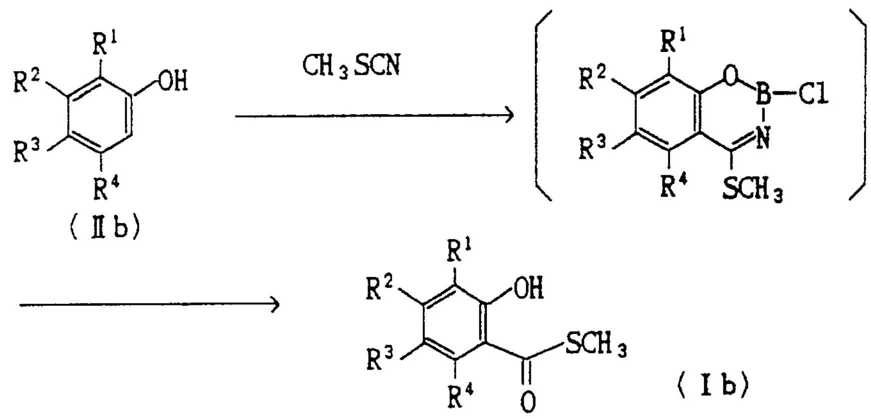


(IIb)

(Ib)

The reaction was performed with boron trichloride and aluminium chloride at room temperature. Other reaction conditions as well as starting materials and end results are shown in Table 6.

Table 6

| Ex. No. | $R^1$ | $R^2$ | $R^3$ | $R^4$ | Solvent | Time (hr) | m.p. (°C) | Yield (%) |
|---|---|---|---|---|---|---|---|---|
| 48 | H | H | OMe | H | BN | 48 | 65 - 66 | 74 |
| 49 | Et | H | H | OMe | DE | 16 | 48 - 49 | 48 |
| 50 | H | OMe | H | OMe | BN | 17 | 111 - 113 | 57 |
| 51 | H | OMe | H | $CH_2Ph$ | DE | 14 | 48 - 49 | 48 |

Example 52

To 5.5 ml of a solution of 2.2 M boron trichloride - 1,2-dichloroethane were added a solution of 1.44 g of α-naphthol in 30 ml of 1,2-dichloroethane, as well as 0.82 ml of methyl thiocyanate and 1.33 g of aluminium chloride under ice-cooling. A fter stirring at room temperature for 17 hr., the reaction solution was cooled with ice water and mixed with 15 ml of 2 N HCl and 30 ml of water. Then the mixture was heated at 110 °C on an oil bath under stirring to evaporate 1,2-dichloroethane and further stirred for 24 hr. After cooling, the mixture was mixed with 50 ml of methylene chloride under stirring and filtered to remove insoluble materials. The methylene chloride solution was dried over anhydrous magnesium sulfate and the solvent

was evaporated. The crude crystals were chromatographed on a column of 20 g of silica gel for purification and 1.51 g of the eluate with toluene was recrystallized from ether - n-hexane to give 1.36 g of 1-hydroxy-2-thionaphthoic acid S-methyl ester at 60 - 61 °C as pale yellow crystals.

Yield : 63 %

Example 53

To 5.5 ml of a solution of 2.2 M boron trichloride - 1,2-dichloroethane were added a solution of 1.44 g of β-naphthol in 30 ml of 1,2-dichloroethane, as well as 0.82 ml of methyl thiocyanate and 1.33 g of aluminium chloride under ice-cooling. After stirring at room temperature for 16 hr., the reaction mixture was cooled with ice water and mixed with 10 g of ice and 40 ml of water, heated at 110 °C on an oil bath under stirring to evaporate 1,2-dichloroethane, and further stirred for 24 hr. After cooling, the resultant was mixed with 50 ml of toluene under stirring and filtered to remove insoluble materials. The toluene solution was dried over anhydrous magnesium sulfate and concentrated. The crude crystals were chromatographed on a column of 30 g of silica gel for purification and 1.19 g of the eluate with toluene was recrystallized from ether - n-hexane to give 1.10 g of 2-hydroxy-1-thionaphthoic acid S-methyl ester melting at 111 - 112 °C as crystals.

Yield : 51 %

## Claims

1. A process for introducing a thiocarboxylic ester group at the ortho-position of phenols or phenylamines, characterised by reacting a phenyl compound having a hydroxy group or an optionally substituted amino or cyclic amino group, of which at least one ortho-position is vacant, with a $C_1$-$C_5$ alkyl thiocyanate, $C_7$-$C_{13}$ aralkyl thiocyanate or $C_6$-$C_{12}$ aryl thiocyanate in the presence of boron trichloride or tribromide and hydrolysing the resulting boron-containing intermediate product under acidic conditions to give a desired end product according to the overall reaction scheme:

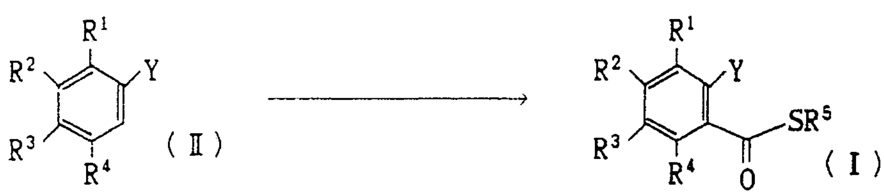

wherein $R^1$,$R^2$,$R^3$ and $R^4$ each are hydrogen, halogen, $C_1$-$C_5$ alkyl, $C_1$-$C_5$ alkoxy, $C_1$-$C_5$ alkylthio, $C_6$-$C_{12}$ aryloxy, $C_7$-$C_{15}$ aralkyl, $C_7$-$C_{15}$ aralkoxy or $C_1$-$C_{10}$ acylamino; or ($R^1$ and $R^2$) or ($R^2$ and $R^3$) taken together form a condensed benzene ring optionally substituted by halogen or $C_1$-$C_5$ alkyl or $C_1$-$C_5$ alkoxy; $R^5$ is $C_1$-$C_5$ alkyl or $C_7$-$C_{13}$ aralkyl or $C_6$-$C_{12}$ aryl; Y is hydroxy, amino or NHR; and R is $C_1$-$C_5$ alkyl, $C_7$-$C_{13}$ aralkyl, $C_6$-$C_{12}$ aryl or N-(methyl- or benzyl-substituted)aza($C_3$-$C_7$)cycloalkyl;or NHRand $R^1$ taken together form a 5- or 6-membered heterocyclic ring B condensed to the initial benzene ring A and represented by the formula:

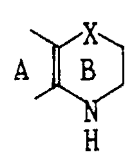

(IV)

in which X is a single bond or $CH_2$, O, S or N(methyl)- and the B ring may optionally be condensed with a benzene, pyridine or cyclohexane ring which may optionally be substituted by halogen or $C_1$-$C_5$

alkyl or $C_1$-$C_5$ alkoxy; said alkyl,alkoxy,aryl, arylkoxy, aralkyl and aralkoxy radicals being optionally substituted by $C_1$-$C_5$ alkyl, $C_1$-$C_5$ alkoxy or halogen

2. A process according to claim 1, in which the starting phenyl compound is represented by the formula:

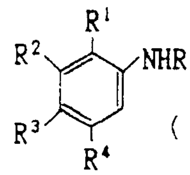
( IIa )

wherein R is $C_1$-$C_5$ alkyl, $C_7$-$C_{15}$ aralkyl, $C_6$-$C_{12}$ aryl or N-(methyl-or benzyl-substituted)aza($C_3$-$C_7$)-cycloalkyl and $R^1$, $R^2$,$R^3$ and $R^4$ are as defined in claim 1.

3. A process according to claim 1, in which the starting phenyl compound is represented by the formula:

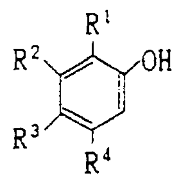
( IIb)

wherein $R^1$,$R^2$,$R^3$ and $R^4$ are as defined in claim 1.

4. A process according to claim 1, in which the starting phenyl compound is represented by the formula:

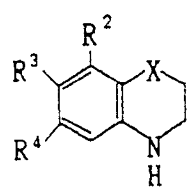
( IIc)

wherein $R^2$,$R^3$,$R^4$ and X are as defined in claim 1.

5. A process according to claim 1, in which the starting phenyl compound is represented by the formula:

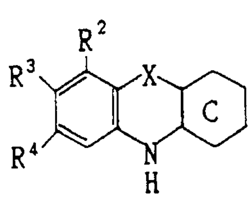
( IId)

wherein the ring C is a benzene or pyridine or cyclohexane ring optionally substituted by halogen or $C_1$-$C_5$ alkyl or $C_1$-$C_5$ alkoxy and $R^2$,$R^3$,$R^4$ and X are as defined in claim 1.

6. A process according to claim 3, in which the compound (IIb) is α-naphthol or β-naphthol.

7. A process according to claim 4, in which the compound (IIc) is 1,2,3,4-tetrahydroquinoline, indoline, 2,3-dihydro-4H-1,4-benzothiazine, N-methylbenzopiperazine or 3,4-dihydro-2H-1,4-benzoxazine.

8. A process according to claim 5, in which the compound (IId) is carbazole or phenothiazine.

9. A process according to claims 3, in which the reaction is carried out in the presence of boron trichloride or tribromide and another Lewis acid selected from aluminium chloride, tin tetrachloride and titanium tetrachloride.

## Revendications

1. Procédé pour l'introduction d'un groupe ester thiocarboxylique en position ortho de phénols ou de phénylamines, caractérisé en ce qu'on fait réagir un composé phényle contenant un groupe hydroxy ou un groupe amino, ou amino cyclique, éventuellement substitué, dont au moins une position ortho est libre, au moyen d'un $C_1$-$C_5$ alkyle thiocyanate, d'un $C_7$-$C_{13}$ aralkyle thiocyanate ou d'un $C_6$-$C_{12}$ aryle thiocyanate en présence de trichlorure ou de tribromure de bore, puis on hydrolyse le produit intermédiaire obtenu, contenant du bore, en milieu acide pour obtenir le produit final recherché selon le processus réactionnel global suivant :

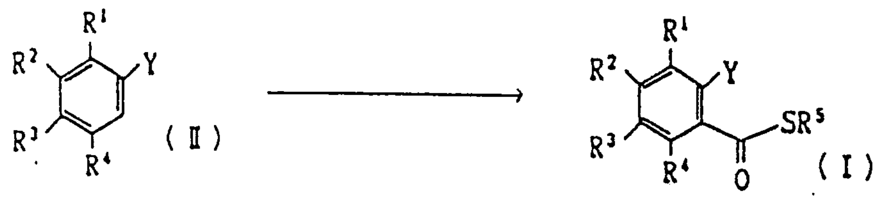
(II) (I)

dans lequel $R^1$, $R^2$, $R^3$ et $R^4$ représentent chacun l'hydrogène,un halogène, un $C_1$-$C_5$ alkyle, un!, $C_1$-$C_5$ alcoxy, un $C_1$-$C_5$ alkylthio, un $C_6$-$C_{12}$ aryloxy, un $C_7$-$C_{15}$ aralkyle, un $C_7$-$C_{15}$ aralcoxy ou un $C_1$-$C_{10}$ acylamino, ou ($R^1$ et $R^2$) ou ($R^2$ et $R^3$) forment ensemble un composé cyclique benzénique condensé, éventuellement substitué par l'halogène ou un $C_1$-$C_5$ alkyle ou un $C_1$-$C_5$ alcoxy, $R^5$ représente un $C_1$-$C_5$ alkyle, un $C_7$-$C_{13}$ aralkyle ou un $C_6$-$C_{12}$ aryle, Y représente un hydroxy, un amino ou NHR et R représente un $C_1$-$C_5$ alkyle, un $C_7$-$C_{13}$ aralkyle, un $C_6$-$C_{12}$ aryle ou un N-aza(substitué par du méthyle ou du benzyle)-($C_3$-$C_7$)cycloalkyle, ou NHR et $R^1$ forment ensemble un composé hétérocyclique B à 5 ou 6 chaînons condensé avec le composé cyclique benzénique initial A et est représenté par la formule suivante :

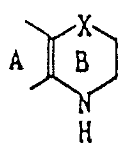
(IV)

dans laquelle X représente une liaison simple ou $CH_2$, O, S ou N(méthyl)- et le composé cyclique B peut éventuellement être condensé avec un composé cyclique de benzène, de pyridine ou de cyclohexane qui peut être éventuellement substitué par l'halogène ou un $C_1$-$C_5$ alkyle ou un $C_1$-$C_5$ alcoxy, lesdits radicaux alkyle, alcoxy, aryle, aryloxy, aralkyle et aralcoxy étant éventuellement substitués par un $C_1$-$C_5$ alkyle, un $C_1$-$C_5$ alcoxy ou l'halogène.

2. Procédé selon la revendication 1, caractérisé en ce que le composé phényle de départ répond à la formule suivante:

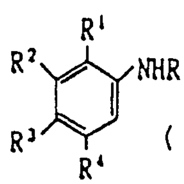
(IIa)

dans laquelle R représente un $C_1$-$C_5$ alkyle, un $C_7$-$C_{15}$ aralkyle, un $C_6$-$C_{12}$ aryle ou un N-aza(substitué par du méthyle ou benzyle)-($C_3$-$C_7$)cycloalkyle et $R^1$, $R^2$, $R^3$ et $R^4$ sont définis comme dans la revendication 1.

3. Procédé selon la revendication 1, caractérisé en ce que le composé phényle de départ répond à la formule suivante :

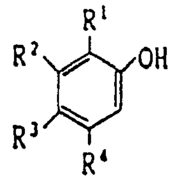

( IIb)

dans laquelle $R^1$, $R^2$, $R^3$ et $R^4$ sont définis comme dans la revendication 1.

4. Procédé selon la revendication 1, caractérisé en ce que le composé phényle de départ répond à la formule suivante :

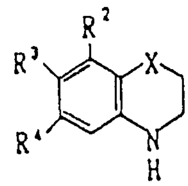

( IIc)

dans laquelle $R^{2+}$, $R^3$, $R^4$ et X sont définis comme dans la revendication 1.

5. Procédé selon la revendication 1, caractérisé en ce que le composé phényle de départ répond à la formule suivante :

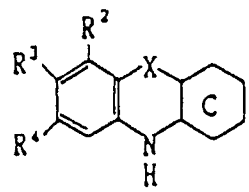

( IId)

dans laquelle le composé cyclique C est un cycle de benzène, de pyridine ou de cyclohexane éventuellement substitué par l'halogène ou un $C_1$-$C_5$ alkyle ou un $C_1$-$C_5$ alcoxy et $R^2$, $R^3$, $R^4$ et X sont définis comme dans la revendication 1.

6. Procédé selon la revendication 3, caractérisé en ce que le composé (IIb) est l'$\alpha$-naphtol ou le $\beta$-naphtol.

7. Procédé selon la revendication 4, caractérisé en ce que le composé (IIc) est la 1,2,3,4-tétrahydroquinoline, l'indoline, la 2,3-dihydro-4H-1,4-benzothiazine, la N-méthylbenzopipérazine ou la 3,4-dihydro-2H-1,4-benzoxazine.

8. Procédé selon la revendication 5, caractérisé en ce que le composé (IId) est le carbazol ou la phénothiazine.

9. Procédé selon la revendication 3, caractérisé en ce que la réaction est réalisée en présence de trichlorure ou de tribromure de bore et d'un autre acide de Lewis choisi parmi le chlorure d'aluminium, le tétrachlorure d'étain et le tétrachlorure de titane.

## Ansprüche

1. Verfahren zur Einführung einer Thiocarboxylestergruppe in die ortho-Stellung von Phenolen oder Phenylaminen, dadurch **gekennzeichnet**, daß eine Phenylverbindung mit einer Hydroxygruppe oder einer gegebenenfalls substituierten Amino- oder cyclischen Aminogruppe, bei der mindestens eine

ortho-Stellung unbesetzt ist, mit einem $C_1$-$C_5$-Alkylthiocyanat, $C_7$-$C_{13}$-Aralkylthiocyanat oder $C_6$-$C_{12}$-Arylthiocyanat in Anwesenheit von Bortrichlorid oder -tribromid umgesetzt wird und das entstehende, Bor enthaltende Zwischenprodukt unter sauren Bedingungen unter Bildung des gewünschten Endprodukts gemäß dem Gesamt-Reaktionsschema hydrolysiert wird:

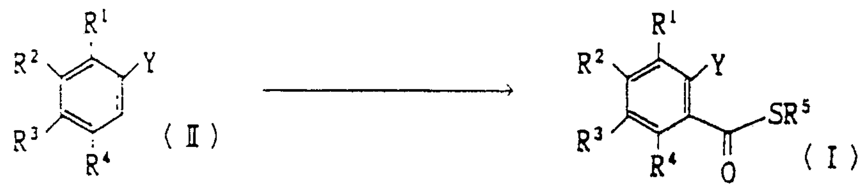

( II ) ( I )

worin $R^1$, $R^2$, $R^3$ und $R^4$ je Wasserstoff, Halogen, $C_1$-$C_5$-Alkyl, $C_1$-$C_5$-Alkoxy, $C_1$-$C_5$-Alkylthio, $C_6$-$C_{12}$-Aryloxy, $C_7$-$C_{15}$-Aralkyl, $C_7$-$C_{15}$-Aralkoxy oder $C_1$-$C_{10}$-Acylamino bedeuten; oder ($R^1$ und $R^2$) oder ($R^2$ und $R^3$) zusammen einen kondensierten Benzolring bilden, der gegebenenfalls durch Halogen oder $C_1$-$C_5$-Alkyl oder $C_1$-$C_5$-Alkoxy substituiert sein kann; $R^5$ $C_1$-$C_5$-Alkyl oder $C_7$-$C_{13}$-Aralkyl oder $C_6$-$C_{12}$-Aryl bedeutet; Y Hydroxy, Amino oder NHR bedeutet; und R $C_1$-$C_5$-Alkyl, $C_7$-$C_{13}$-Aralkyl, $C_6$-$C_{12}$-Aryl oder N-(Methyl- oder -Benzyl-substituiertes)-aza-($C_3$-$C_7$)-cycloalkyl bedeutet; oder NHR und $R^1$ zusammen einen 5- oder 6gliedrigen heterocyclischen Ring B bilden, der an dem AnfangsBenzolring A kondensiert ist und durch die Formel:

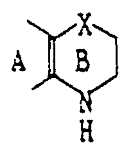

(IV)

dargestellt wird, worin X eine Einfachbindung oder $CH_2$, O, S oder N-(Methyl)- bedeutet und der Ring B gegebenenfalls mit einem Benzol-, Pyridin- oder Cyclohexanring kondensiert sein kann, welcher gegebenenfalls durch Halogen oder $C_1$-$C_5$-Alkyl oder $C_1$-$C_5$-Alkoxy substituiert sein kann, wobei die Alkyl-, Alkoxy-, Aryl-, Aryloxy-, Aralkyl- und Aralkoxygruppen gegebenenfalls durch $C_1$-$C_5$-Alkyl, $C_1$-$C_5$-Alkoxy oder Halogen substituiert sein können.

2. Verfahren nach Anspruch 1, dadurch **gekennzeichnet**, daß die als Ausgangsmaterial verwendete Phenylverbindung durch die Formel:

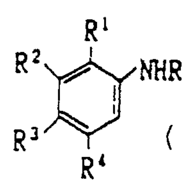

( IIa )

dargestellt wird, worin R $C_1$-$C_5$-Alkyl, $C_7$-$C_{15}$-Aralkyl, $C_6$-$C_{12}$-Aryl oder N-(Methyl- oder -Benzyl-substituiertes)-aza-($C_3$-$C_7$)-cycloalkyl bedeutet und $R^1$, $R^2$, $R^3$ und $R^4$ die in Anspruch 1 gegebenen Bedeutungen besitzen.

3. Verfahren nach Anspruch 1, dadurch **gekennzeichnet**, daß die als Ausgangsmaterial verwendete Phenylverbindung durch die Formel:

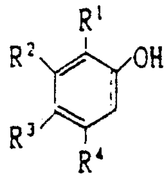

(IIb)

worin $R_1$, $R^2$, $R^3$ und $R^4$ die in Anspruch 1 gegebenen Bedeutungen besitzen, dargestellt wird.

4. Verfahren nach Anspruch 1, dadurch **gekennzeichnet**, daß die als Ausgangsmaterial verwendete Phenylverbindung durch die Formel:

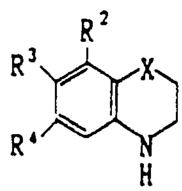

(IIc)

dargestellt wird, worin $R^2$, $R^3$, $R^4$ und X die in Anspruch 1 gegebenen Bedeutungen besitzen.

5. Verfahren nach Anspruch 1, dadurch **gekennzeichnet,** daß die als Ausgangsmaterial verwendete Phenylverbindung durch die Formel:

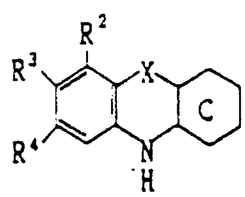

(IId)

dargestellt wird, worin der Ring C ein Benzol- oder Pyridinoder Cyclohexanring ist, der gegebenenfalls durch Halogen oder $C_1$-$C_5$-Alkyl oder $C_1$-$C_5$-Alkoxy substituiert sein kann, und worin $R^2$, $R^3$, $R^4$ und X die in Anspruch 1 gegebenen Bedeutungen besitzen.

6. Verfahren nach Anspruch 3, dadurch **gekennzeichnet,** daß die Verbindung (IIb) α-Naphthol oder β-Naphthol ist.

7. Verfahren nach Anspruch 4, dadurch **gekennzeichnet,** daß die Verbindung (IIc) 1,2,3,4-Tetra-hydrochinolin, Indolin, 2,3-Dihydro-4H-1,4-benzothiazin, N-Methylbenzopiperazin oder 3,4-Dihydro-2H-1,4-benzoxazin ist.

8. Verfahren nach Anspruch 5, dadurch **gekennzeichnet**, daß die Verbindung (IId) Carbazol oder Phenothiazin ist.

9. Verfahren nach Anspruch 3, dadurch **gekennzeichnet,** daß die Reaktion in Anwesenheit von Bortrichlorid oder -tribromid und einer anderen Lewis-Säure, ausgewählt unter Aluminiumchlorid, Zinntetrachlorid und Titantetrachlorid, durchgeführt wird.